Europäisches Patentamt

European Patent Office

Office européen des brevets

(19)

(11) Veröffentlichungsnummer: **0 378 127 B1**

## (12) EUROPÄISCHE PATENTSCHRIFT

(45) Veröffentlichungstag der Patentschrift :
02.10.91 Patentblatt 91/40

(51) Int. Cl.⁵ : **C07H 7/033, C11D 1/08, A23L 1/30**

(21) Anmeldenummer : 90100213.9

(22) Anmeldetag : 05.01.90

(54) Verfahren zur Herstellung eines Gemisches von Oxidationsprodukten der Saccharose und deren Verwendung.

(30) Priorität : 12.01.89 DE 3900677

(43) Veröffentlichungstag der Anmeldung :
18.07.90 Patentblatt 90/29

(45) Bekanntmachung des Hinweises auf die
Patenterteilung :
02.10.91 Patentblatt 91/40

(84) Benannte Vertragsstaaten :
AT BE CH DE DK FR GB IT LI NL SE

(56) Entgegenhaltungen :
EP-A- 0 218 150
DE-A- 886 305

(73) Patentinhaber : HOECHST
AKTIENGESELLSCHAFT
Postfach 80 03 20
W-6230 Frankfurt am Main 80 (DE)

(72) Erfinder : Leupold, Ernst Ingo, Dr.
Auf der Erlenwiese 61
W-6392 Neu-Anspach (DE)
Erfinder : Schönwälder, Karl-Heinz, Dr.
Am Feldbergblick 6
W-6233 Kelkheim(Taunus) (DE)
Erfinder : Fritsche-Lang, Wolfram, Dr.
Rhönstrasse 7
W-6140 Bensheim (DE)
Erfinder : Schlingmann, Merten, Prof. Dr.
Schneidhainer Strasse 32a
W-6240 Königstein/Taunus (DE)
Erfinder : Linkies, Adolf, Dr.
Loreleistrasse 12
W-6230 Frankfurt am Main 80 (DE)
Erfinder : Gohla, Werner
Rathausstrasse 73
W-5216 Niederkassel (DE)
Erfinder : Dany, Franz-Josef, Dr.
Heddinghovenerstrasse 47
W-5042 Erftstadt (DE)

## Beschreibung

Die vorliegende Erfindung betrifft ein Verfahren zur Herstellung eines im wesentlichen aus dem Natriumsalz der Saccharose-tricarbonsäure (STA) bestehenden Gemisches von Oxidationsprodukten der Saccharose und deren Verwendung, insbesondere als Zusatzstoff in Waschmitteln.

Aufgrund ihrer gewässereutrophierenden Wirkung ist der Einsatz von Phosphaten in Wasch- und Reinigungsmitteln in einer Reihe von Ländern gesetzlich beschränkt, zum Teil sogar verboten. Daher sind inzwischen eine Vielzahl von Ersatzstoffen für die Phosphate, insbesondere für das Natriumtripolyphosphat, als "Builder" (Gerüststoff) entwickelt und vorgeschlagen worden. In Summe werden jedoch die wünschenswerten, waschtechnischen Eigenschaften des Natriumtripolyphosphates bisher von keinem Einzelstoff erreicht. Lediglich Gerüststoffkombinationen sind in der Lage, in erster Näherung die Phosphate zu ersetzen. Nur relativ wenige Phosphatersatzstoffe — oder besser gesagt Phosphatteilersatzstoffe — sind hinsichtlich ihrer ökologischen Eigenschaften voll befriedigend. Wenn sie die Gewässereutrophie auch nicht fördern, so zeigen sie zum Teil aber ein Umweltverhalten, das als bedenklich angesehen werden muß, z.B. die Remobilisierung von Schwermetallen aus den Gewässersedimenten oder mangelnde biologische Abbaubarkeit, so daß ihre Umweltrelevanz, auch wenn sie zunächst einmal nach heutigem Kenntnisstand nicht als toxisch einzustufen sind, ungewiß ist. Die Suche nach effektiven Waschmittelbuildern, die bezüglich ihres ökologischen Verhaltens als unbedenklich eingestuft werden können, geht daher weiter.

Aus der DE-OS 3535720 ist ein Verfahren zur Herstellung der Saccharose-tricarbonsäure bekannt. Danach wird Saccharose mit Sauerstoff, gegebenenfalls im Gemisch mit inerten Gasen, z.B. in Form von Luft, mit Hilfe eines wesentlichen effektiveren Katalysators als Platin/Tonerde in einem wäßrigen Medium oxidiert. Die so gebildete Saccharose-tricarbonsäure kann dabei als solche oder in Form der unmittelbar anfallenden Reaktionsprodukte — das sind insbesondere Gemische dieser Säure mit verschiedenen Mono- und/oder Dicarbonsäuren sowie Neben- oder Abbauprodukten, wie z.B. Oxalsäure — in Waschmittelformulierungen als Buildersubstanz eingesetzt werden. Es hat sich jedoch gezeigt, daß sich diese Produkte zwar durch ein gutes ökologisches Verhalten auszeichnen, im Hinblick auf ihr waschtechnisches Verhalten aber nur eine eingeschränkte Effektivität aufweisen.

Überraschenderweise wurde nun gefunden, daß das Rohgemisch der nach dem bekannten Verfahren erhaltenen Reaktionsprodukte wesentlich verbesserte waschtechnische Eigenschaften aufweist, wenn der Verfahrensablauf in charakteristischer Weise abgeändert wird.

Die Erfindung betrifft daher ein Verfahren zur Herstellung eines ein Salz, vorzugsweise das Natriumsalz, der Saccharose-tricarbonsäure enthaltendes Gemisch von Oxidationsprodukten der Saccharose, welches erhalten wird durch Umsetzung von Saccharose mit Sauerstoff in einem wäßrigen Medium in Gegenwart eines Platinmetall-Aktivkohle-Katalysators bei erhöhten Temperaturen und Neutralisation des Umsetzungsproduktes zur Überführung in die Salzform mit vorzugsweise einer alkalischen Natriumverbindung, dadurch gekennzeichnet, daß man die Umsetzung diskontinuierlich durchführt, die Ausgangsprodukte im Verlauf von mehreren Stunden von Raumtemperatur bis auf 60 bis 95°C, vorzugsweise 70 bis 80°C, schrittweise erwärmt und dann das Umsetzungsprodukt gegebenenfalls isoliert.

Weiterhin bezieht sich die Erfindung auf die Verwendung dieser Oxidationsprodukte insbesondere als Zusatz in Wasch- und Reinigungsmitteln.

Das erfindungsgemäße Verfahren wird grundsätzlich wie das Verfahren gemäß der genannten DE-OS 3535720 durchgeführt, jedoch mit den aus dem obigen Kennzeichen ersichtlichen Maßnahmen.

Erfindungsgemäß wird also Saccharose mit Sauerstoff, gegebenenfalls im Gemisch mit inerten Gasen wie Stickstoff, z.B. also in Form von Luft, in wäßrigem Medium in Gegenwart von geeigneten Katalysatoren oxidiert.

Als geeignete Katalysatoren kommen solche auf Basis von Platinmetallen in Frage, wie Osmium, Iridium, Rhodium, Ruthenium, Palladium und/oder Platin, wobei Platin bevorzugt ist, die auf Aktivkohlen als Träger aufgebracht sind. Diese Katalysatoren enthalten im allgemeinen 5 bis 10 Gew.-% Metall, insbesondere Platin. Bevorzugt erfolgt dabei die Oxidation in der Weise, daß fester Katalysator in wäßrigem Reaktionsmedium mit gasförmigem Sauerstoff behandelt wird, d.h. in einer Dreiphasenreaktion. Nach einer weiteren bevorzugten Ausführungsform wird hochkonzentrierter Sauerstoff verwendet und die Reaktionslösung im Kreislauf geführt, was die Einstellung bzw. das Konstanthalten des pH-Wertes erleichtern kann.

Das erfindungsgemäße Verfahren wird in der Regel diskontinuierlich durchgeführt, beispielsweie in einem Blasensäulenreaktor. Prinzipiell könnte die Umsetzung auch kontinuierlich gestaltet werden, wenn eine Einrichtung mit mehreren Kaskaden mit jeweils steigenden Temperaturen verwendet wird. Wegen des erheblichen apparativen Aufwands ist jedoch die kontinuierliche Umsetzung weit weniger wirtschaftlich.

Im allgemeinen arbeitet man bei dem erfindungsgemäßen Verfahren bei Atmosphärendruck, jedoch ist auch die Anwendung von Gesamtdrucken bis zu 100 bar, vorzugsweise 10 bar, möglich, wodurch das Angebot von Sauerstoff und/oder die Reaktionstemperatur erhöht werden kann. Die Einhaltung bestimmter Saccharose-Konzentrationen ist dabei vorteilhaft ; unter 5 Gew.-% findet leicht eine Überoxidation statt und über 20 Gew.-% lassen sich bei Atmosphärendruck nur verhältnismäßig niedrige Umsätze erzielen. Der pH-Wert der zu oxidierenden Saccharose-Lösung wird in der Regel zwischen 5 und 9, vorzugsweise zwischen 6 und 8,5 und insbesondere zwischen 7 und 8 gehalten. Dies geschieht zweckmäßigerweise mit einer alkalischen, vorzugsweise physiologisch verträglichen Verbindung eines Metalls der I. und II. Hauptgruppe des Periodensystems, vorzugsweise eines Alkalimetalls, wie Natrium und/oder Kalium, insbesondere Natrium. Hierfür geeignet sind entsprechende Puffersubstanzen, wie Natrium(hydrogen)carbonat oder entsprechende Basen, wie Natronlauge.

Die Umsetzung kann in allen Apparaturen, die sich für die Durchführung von Reaktionen in der flüssigen Phase mit oder ohne Anwendung von Überdruck eignen, durchgeführt werden, also beispielsweise in einem doppelmanteligen Blasensäulenreaktor ausgeführt werden, in dem sich die Suspension des Katalysators in dem wäßrigen Medium befindet und der unten eine Fritte oder eine geeignete andere poröse Membran enthält und von der Unterseite mit einem durch diese Trennmembran feinstverteilten Gasstrom durchströmt wird. Aus wirtschaftlichen Gründen wird der Sauerstoff zweckmäßig mit einer solchen Geschwindigkeit durch das Reaktionsmedium geleitet, daß der katalytisch aktivierte Sauerstoff am oberen Ende der Blasensäule eben verbraucht ist. Zur Verbesserung der Durchmischung und Verlängerung der Einwirkungszeit des Sauerstoffs kann es vorteilhaft sein, das Reaktionsgemisch zu rühren. Eine andere geeignete Apparatur ist zum Beispiel der Rieselphasenreaktor.

Ein erfindungswesentliches Merkmal ist die spezielle Temperaturführung bei der Oxidation der Saccharose in der Weise, daß die Temperatur im Verlauf von mehreren Stunden, vorzugsweise 5 bis 15, insbesondere 8 bis 12 Stunden, von Raumtemperatur auf Temperaturen von ca. 60 bis 95°C, vorzugsweise 70 bis 80°C kontinuierlich oder diskontinuierlich gesteigert wird.

Gemäß einer bevorzugten Ausgestaltungsform wird dabei die Temperatur der Ausgangsprodukte, ausgehend von einer Raumtemperatur von beispielsweise 20°C, im Verlaufe von ca. 5 bis 15, vorzugsweise von 8 bis 12 und insbesondere ca. 10 Stunden bis auf ca. 70 bis 80°C erhöht, wobei man die Temperatur alle 5 bis 20, vorzugsweise alle 8 bis 15 und insbesondere alle 12 Minuten um jeweils 0,5 bis 3°C, vorzugweise um etwa 1°C steigert.

Die erforderliche Reaktionszeit wird zweckmäßig dadurch ermittelt, daß man in gewissen Zeitabständen Proben der Reaktionslösung entnimmt und analysiert. Beispielsweise kann die Ausbeute der Reaktionsprodukte auf einfache Weise durch Analyse einer Probe mit Hilfe der Hochdruckflüssigkeitschromatographie im Vergleich zu Standardlösungen laufend bestimmt werden. Die Optimierung der Reaktionszeit ist zu empfehlen, da eine unnötig verlängerte Einleitung von Sauerstoff zu Überoxidationen und Verminderung der Ausbeute an den gewünschten Reaktionsprodukten führen kann.

Die anfallende Reaktionslösung, in der die gebildete Saccharose im Gemisch mit weniger oxidierten Vorstufen sowie verschiedenen Neben- oder Abbauprodukten vorliegt, wird dann anschließend — zur Überführung in die Salzform — auf einen pH-Wert von 8 bis 10, vorzugsweise ungefähr 9, eingestellt, falls dieser pH-Wert nach der Oxidation nicht ohnehin schon gegeben ist. Dies kann durch Zugabe entsprechender Mengen der weiter oben beschriebenen alkalischen Verbindungen, vorzugsweise einer alkalischen Natriumverbindung, wie Natronlauge oder Natrium(hydrogen)carbonat, geschehen.

Das erfindungsgemäß erhältliche Gemisch von Oxidationsprodukten wird dann aus der Reaktionslösung in bekannter Weise aufgearbeitet bzw. isoliert, z.B. durch Gefrier- oder Sprühtrocknung oder durch andere hierfür geeignete Verfahren. In der so erhaltenen festen Mischung bildet das Salz der Saccharose-Tricarbonsäure den Hauptbestandteil und beträgt im allgemeinen mindestens 30 Gew.-%, vorzugsweise mehr als 40 Gew.-% dieser Mischung.

Das so isolierte Produkt eignet sich vor allem als Zusatzstoff (Buildersubstanz) in Waschmittel- oder Reinigungsmittel-Formulierungen. Daneben ist es auch als Lebensmittelzusatzstoff, als Vernetzer in Lackzubereitungen etc. einsetzbar.

Die nachfolgenden Beispiele erläutern die Erfindung.

**Beispiele**

**I. Herstellung der Saccharose-tricarbonsäure**

In ein von außen beheiztes, senkrecht angeordnetes Glasrohr (Durchmesser : 50 mm, Länge : 800 mm), das mit einer Mischung von 120 g Saccharose, 1,2 l Wasser und 50 g eines handelsüblichen Katalysators (5% Platin auf Aktivkohle) gefüllt war, leitete man von unten durch eine Glasfritte $5,6 \times 10^{-6} m_n^3/s$ (20 NL/h) Sauerstoff. Die Temperatur betrug am Anfang 20°C und wurde im Verlauf von 10 h alle 12 Minuten um jeweils 1°C bis auf 70°C erhitzt. Durch Zugabe von 30 %iger wäßriger NaOH wurde der pH-Wert der Lösung bei 8,0 gehalten und am Ende der Rekation auf 9,0 eingestellt. Das filtrierte Reaktionsgemisch wurde sprühgetrocknet, Ausbeute : 114 g. Der Gehalt an Saccharose-tricarbonsäure in Form des Trinatriumsalzes betrug 41 Gew.-%.

## II. Waschmitteltechnische Prüfung

Zur Charakterisierung der erfindungsgemäß erhältlichen Umsetzungsprodukte als Buildersubstanz wurden folgende Kenndaten bestimmt :
— Kalkbindevermögen : ca. 40 mg Ca/g
— Biologische Abbaubarkeit (statischer Test nach DIN 38.412/25) :  >  70%
— Bakterientoxizität (Zehrungstest) :  >  1.000 mg/l

Die Überlegenheit des erfindungsgemäß erhaltenen Gemisches als Buildersubstanz zeigt sich in den Prüfergebnissen von Waschmittelformulierungen, in denen lediglich die nach der DE-OS 3535720 erhaltene Saccharose-tricarbonsäure durch das erfindungsgemäße Produkt substituiert wurde. Die waschtechnische Prüfung dieser Waschmittel erfolgte nach den anerkannten Regeln der Technik in Anlehnung an DIN 44.983:
— Die Waschkraft (Remissionsdifferenz) wurde durch Remissionsmessung (Farbmeßgerät RFC 3 der Fa. Zeiss) an WFK-und EMPA-Test-Schmutzgewebe photometrisch ermittelt. Hierbei wurde die "Differenzmethode" entsprechend folgender Gleichung angewendet :

$$\% \, \triangle R = \% \, R_g - \% \, R_u$$

$\% \, \Delta R =$     % Remissionsdifferenz (Waschkraft)
$\% \, R_g =$     % Remission des gewaschenen Gewebes
$\% \, R_u =$     % Remission des ungewaschenen Gewebes

— Die Gewebeablagerung (Inkrustation) wurde in Form der anorganischen Gewebeasche als prozentualer Glührückstand bei 800°C bestimmt.

Die Herstellung der Waschmittelpulver wurde teils nach dem sogenannten Heißsprühverfahren und teils nach dem sogenannten Sprühnebelmischverfahren (Trockenmischprozeß) durchgeführt.

Die Heißsprühung erfolgte mittels eines Laborsprühtrockners (Fa. Büchi, Typ 190) unter folgenden Bedingungen :
— Eingangstemperatur : ca. 180°C
— Ausgangstemperatur : ca. 100°C
— Sprühdruck : 5 bar
— Feststoffkonzentration : 30 Gew%

Bei dem Sprühnebelmischverfahren kam ein Freifallmischer zur Anwendung, wobei die flüssigen Bestandteile mittels einer geeigneten Sprühvorrichtung aufgedüst wurden. Wie im einzelnen verfahren wurde, ist in "Seifen, Fette, Öle, Wachse", 99. Jahrgang, Nr. 13, 1973, Seiten 351 bis 357 beschrieben.

# Waschmittelformulierungen

| | Sprühnebelmisch-verfahren | | Heißsprüh-verfahren | | Sprühnebelmisch-verfahren | |
|---|---|---|---|---|---|---|
| | Beispiel | | Beispiel | | Beispiel | |
| | 1 A | 1 B | 2 A | 2 B | 3 A | 3 B |
| **Tricarbonsäure (STA)** | | | | | | |
| STA (bekanntes Produkt) | 10,0 | - | 10,0 | - | 21,5 | - |
| STA (erfindungsgemäß) | - | 10,0 | - | 10,0 | - | 21,5 |
| Zeolith | 21,5 | 21,5 | 21,5 | 21,5 | 10,0 | 10,0 |
| Na-Perborat-tetrahydrat | 20,0 | 20,0 | 20,0 | 20,0 | 20,0 | 20,0 |
| Anionische Tenside | 7,0 | 7,0 | 7,0 | 7,0 | 7,0 | 7,0 |
| Nichtionische Tenside | 4,0 | 4,0 | 4,0 | 4,0 | 4,0 | 4,0 |
| Seife | 3,5 | 3,5 | 3,5 | 3,5 | 3,5 | 3,5 |
| Na-Silicat | 5,0 | 5,0 | 5,0 | 5,0 | 5,0 | 5,0 |
| Carboxymethylcellulose | 1,0 | 1,0 | 1,0 | 1,0 | 1,0 | 1,0 |
| Methylcellulose | 0,5 | 0,5 | 0,5 | 0,5 | 0,5 | 0,5 |
| *Rest übliche Waschmittelbestandteile auf 100 %* | | | | | | |

EP 0 378 127 B1

| Waschmittelformulierungen | | |
|---|---|---|
| | **Heißsprüh-verfahren** | **Sprühnebelmisch-verfahren** |
| | **Beispiel 4** | **Beispiel 5** |
| **Tricarbonsäure (STA)** | | |
| STA (bekanntes Produkt) | - | - |
| STA (erfindungsgemäß) | - | - |
| Zeolith | 10,0 | 21,5 |
| Na-Perborat-tetrahydrat | 20,0 | 20,0 |
| Anionische Tenside | 7,0 | 7,0 |
| Nichtionische Tenside | 4,0 | 4,0 |
| Seife | 3,5 | 3,5 |
| Na-Silicat | 5,0 | 5,0 |
| Carboxymethylcellulose | 1,0 | 1,0 |
| Methylcellulose | 0,5 | 0,5 |
| | *Rest übliche Waschmittelbestandteile auf 100 %* | |

EP 0 378 127 B1

| Waschversuche nach DIN 44983 (Zwellaugenverfahren, 90 °C - Wäsche, Wasserhärte 18 °d) | | | | |
|---|---|---|---|---|
| | Dosierung (g) | % Asche nach 25 Waschzyklen | | |
| Beispiel | Vor- / Klar-wäsche | Frottee (Vossen) | Baumwolle (EMPA) | Doppelripp (WFK) |
| 1 A | 150 / 150 | 2,1 | 2,3 | 2,4 |
| 1 B | 150 / 150 | 1,2 | 0,6 | 0,6 |
| 2 A | 150 / 150 | 2,2 | 2,4 | 2,6 |
| 2 B | 150 / 150 | 1,3 | 0,4 | 0,4 |
| 3 A | 150 / 150 | 1,6 | 1,4 | 1,9 |
| 3 B | 150 / 150 | 0,3 | 0,4 | 0,6 |
| 4 | 150 / 150 | 3,5 | 3,3 | 3,2 |
| 5 | 150 / 150 | 5,9 | 5,1 | 4,8 |

EP 0 378 127 B1

EP 0 378 127 B1

| Waschversuche nach DIN 44983 (Zwellaugenverfahren, 60 °C - Wäsche, Wasserhärte 18 °d) | | | | |
|---|---|---|---|---|
| **Beispiel** | **Dosierung (g)** Vor- / Klar- wäsche | **Primärwaschwirkung an verschiedenen Testgeweben** ( % Remissionsdifferenz ) | | |
| | | EMPA Baumwolle (101) | WFK Baumwolle (10 C) | WFK Baumwolle (10 D) |
| 1 A | 150 / 150 | 26 | 21 | 23 |
| 1 B | 150 / 150 | 28 | 24 | 26 |
| 2 A | 150 / 150 | 25 | 22 | 22 |
| 2 B | 150 / 150 | 27 | 23 | 25 |
| 3 A | 150 / 150 | 27 | 25 | 26 |
| 3 B | 150 / 150 | 30 | 29 | 28 |
| 4 | 150 / 150 | 20 | 15 | 18 |
| 5 | 150 / 150 | 22 | 18 | 20 |

## Patentansprüche

1. Verfahren zur Herstellung eines ein Salz der Saccharose-tricarbonsäure enthaltendes Gemisch von Oxidationsprodukten der Saccharose, welches erhalten wird durch Umsetzung von Saccharose mit Sauerstoff in einem wäßrigen Medium in Gegenwart eines Platinmetall-Aktivkohle-Katalysators bei erhöhten Temperaturen und Neutralisation des Umsetzungsprodukts zur Überführung in die Salzform, dadurch gekennzeichnet, daß man die Umsetzung diskontinuierlich durchführt, die Ausgangsprodukte im Verlauf von mehreren Stunden von Raumtemperatur bis auf 60 bis 95°C schrittweise erwärmt und dann das Umsetzungsprodukt gegebenenfalls isoliert.

2. Verfahren nach Anspruch 1, dadurch gekennzeichnet, daß die Endtemperatur 70 bis 80°C beträgt.

3. Verfahren nach Anspruch 1 und/oder 2, dadurch gekennzeichnet, daß man die Temperatur der Ausgangsprodukte, ausgehend von Raumtemperatur, im Verlauf von 8 bis 12 Stunden bis auf 70 bis 80°C erhöht, indem man die Temperatur etwa alle 8 bis 15 Minuten um jeweils etwa 1°C steigert.

4. Verfahren nach mindestens einem der Ansprüche 1 bis 3, dadurch gekennzeichnet, daß man vor der Isolierung einen pH-Wert von 8 bis 10, vorzugsweise 9, einstellt.

5. Verfahren nach mindestens einem der Ansprüche 1 bis 4, dadurch gekennzeichnet, daß man die Reaktion in der Weise durchführt, daß fester Katalysator in einem wäßrigen Reaktionsmedium mit gasförmigem, vorzugsweise hochkonzentriertem Sauerstoff behandelt wird.

6. Verfahren nach mindestens einem der Ansprüche 1 bis 5, dadurch gekennzeichnet, daß die Reaktion bei Atmosphärendruck erfolgt.

7. Verfahren nach einem oder mehreren der Ansprüche 1 bis 6, dadurch gekennzeichnet, daß das flüssige Reaktionsmedium 5 bis 20 Gew.-% Saccharose enthält.

8. Verfahren nach einem oder mehreren der Ansprüche 1 bis 7, dadurch gekennzeichnet, daß man die Oxidation bei einem pH-Wert von 5 bis 9, vorzugsweise 6 bis 8,5 durchführt.

9. Verfahren nach einem oder mehreren der Ansprüche 1 bis 8, dadurch gekennzeichnet, daß der Katalysator ein Platin/Aktivkohle-Katalysator ist.

10. Verfahren nach einem oder mehreren der Ansprüche 1 bis 9, dadurch gekennzeichnet, daß der Katalysator 5 bis 10 Gew.-% Metall enthält.

11. Verfahren nach mindestens einem der Ansprüche 1 bis 10, dadurch gekennzeichnet, daß das Salz der Saccharose-tricarbonsäure das Natriumsalz ist.

12. Verfahren nach mindestens einem der Ansprüche 1 bis 11, dadurch gekennzeichnet, daß die Neutralisation mit einer alkalischen Natriumverbindung, vorzugsweise Natronlauge, erfolgt.

13. Verwendung der Reaktionsprodukte gemäß mindestens einem der Ansprüche 1 bis 12 in Wasch- oder Reinigungsmitteln oder als Lebensmittelzusatzstoff.

14. Wasch- oder Reinigungsmittel, enthaltend die Reaktionsprodukte, hergestellt nach mindestens einem der Ansprüche 1 bis 12.

## Claims

1. A process for the preparation of a mixture of sucrose oxidation products which contains a salt of sucrosetricarboxylic acid and which is obtained by reaction of sucrose with oxygen in an aqueous medium in the presence of a platinum metal/active carbon catalyst at elevated temperatures and neutralization of the reaction product to convert into the salt form, which comprises the reaction being carried out discontinuously, the starting materials being heated stepwise over the course of several hours from room temperature up to 60 to 95°C, and then the reaction product being isolated where appropriate.

2. The process as claimed in claim 1, wherein the final temperature is 70 to 80°C.

3. The process as claimed in claim 1 and/or 2, wherein the temperature of the starting materials is raised, starting from room temperature, to 70 to 80°C over the course of 8 to 12 hours by increasing the temperature by about 1°C about every 8 to 15 minutes.

4. The process as claimed in at least one of claims 1 to 3, wherein the pH is adjusted to 8 to 10, preferably 9, before the isolation.

5. The process as claimed in at least one of claims 1 to 4, wherein the reaction is carried out in such a way that the solid catalyst is treated in an aqueous reaction medium with gaseous, preferably highly concentrated oxygen.

6. The process as claimed in at least one of claims 1 to 5, wherein the reaction is carried out under atmos-

9

pheric pressure.

7. The process as claimed in one or more of claims 1 to 6, wherein the liquid reaction medium contains 5 to 20% by weight of sucrose.

8. The process as claimed in one or more of claims 1 to 7, wherein the oxidation is carried out at a pH of 5 to 9, preferably 6 to 8.5.

9. The process as claimed in one or more of claims 1 to 8, wherein the catalyst is a platinum/active carbon catalyst.

10. The process as claimed in one or more of claims 1 to 9, wherein the catalyst contains 5 to 10% by weight of metal.

11. The process as claimed in at least one of claims 1 to 10, wherein the salt of sucrosetricarboxylic acid is the sodium salt.

12. The process as claimed in at least one of claims 1 to 11, wherein the neutralization is carried out with an alkaline sodium compound, preferably sodium hydroxide solution.

13. The use of the reaction products as claimed in at least one of claims 1 to 12 in washing or cleaning agents or as additive to foodstuffs.

14. A washing or cleaning agent containing the reaction products prepared as claimed in at least one of claims 1 to 12.


## Revendications

1. Procédé de préparation d'un mélange de produits d'oxydation du saccharose contenant un sel de l'acide saccharo-tricarboxylique, mélange que l'on obtient en faisant réagir à chaud le saccharose avec de l'oxygène dans un milieu aqueux en présence d'un catalyseur formé d'un métal de la famille du platine sur du charbon actif puis en neutralisant le produit de la réaction pour le salifier, procédé caractérisé en ce que l'on effectue la réaction en discontinu en chauffant progressivement ou par étapes les produits dont on part, en plusieurs heures, depuis la température ambiante jusqu'à 60 à 95°C, puis on isole éventuellement le produit formé.

2. Procédé selon la revendication 1, caractérisé en ce que la température finale est de 70 à 80°C.

3. Procédé selon la revendication 1 et/ou 2, caractérisé en ce que l'on élève la température des produits dont on part depuis la température ambiante, en 8 à 12 heures, jusqu'à 70 à 80°C, en l'élevant d'environ 1°C toutes les 8 à 15 minutes environ.

4. Procédé selon une ou plusieurs des revendications 1 à 3, caractérisé en ce que, avant d'isoler le produit, on règle le pH entre 8 et 10, de préférence à 9.

5. Procédé selon une ou plusieurs des revendications 1 à 4, caractérisé en ce que l'on effectue la réaction en faisant passer de l'oxygène gazeux, de préférence très concentré, sur le catalyseur solide dans un milieu de réaction aqueux.

6. Procédé selon une ou plusieurs des revendications 1 à 5, caractérisé en ce que l'on effectue la réaction à la pression atmosphérique.

7. Procédé selon une ou plusieurs des revendications 1 à 6, caractérisé en ce que le milieu de réaction liquide contient de 5 à 20% en poids de saccharose.

8. Procédé selon une ou plusieurs des revendications 1 à 7, caractérisé en ce que l'on effectue l'oxydation à un pH de 5 à 9, de préférence de 6 à 8,5.

9. Procédé selon une ou plusieurs des revendications 1 à 8, caractérisé en ce que le catalyseur est un catalyseur formé de platine sur du charbon actif.

10. Procédé selon une ou plusieurs des revendications 1 à 9, caractérisé en ce que le catalyseur comprend de 5 à 10% en poids du métal.

11. Procédé selon une ou plusieurs des revendications 1 à 10, caractérisé en ce que le sel de l'acide saccharosetricarboxylique est le sel sodique.

12. Procédé selon une ou plusieurs des revendications 1 à 11, caractérisé en ce que l'on effectue la neutralisation avec un composé alcalin du sodium, de préférence avec une lessive de soude.

13. L'emploi des produits obtenus selon une ou plusieurs des revendications 1 à 12 dans des produits de lavage ou de nettoyage ou comme additifs pour aliments.

14. Produits de lavage ou nettoyage contenant les produits qui ont été obtenus selon une ou plusieurs des revendications 1 à 12.